Europäisches Patentamt

European Patent Office

Office européen des brevets

(11) Publication number: **0 308 031 B1**

## EUROPEAN PATENT SPECIFICATION

(45) Date of publication of patent specification: **28.04.93** (51) Int. Cl.5: **C08F 38/00**, C08F 2/24

(21) Application number: **88202022.5**

(22) Date of filing: **14.09.88**

(54) **Polymeric surfactants.**

(30) Priority: **15.09.87 GB 8721698**

(43) Date of publication of application:
**22.03.89 Bulletin 89/12**

(45) Publication of the grant of the patent:
**28.04.93 Bulletin 93/17**

(84) Designated Contracting States:
**BE DE FR GB IT NL SE**

(56) References cited:

**CHEMICAL ABSTRACTS, vol. 90, no. 12, March 1979, pages 74-75, abstract no. 88909v, Columbus, Ohio, US**

**JOURNAL OF POLYMER SCIENCE: POLYMER SYMPOSIUM 71, 1984, pages 247-257, John Wiley & Sons, Inc., US; G.N. PATEL et al.: "Synthesis and polymerization of water-soluble polydiacetylenes"**

**CHEMICAL ABSTRACTS, vol. 101, no. 18, October 1984, page 87, abstract no. 153588b, Columbus, Ohio, US**

**CHEMICAL ABSTRACTS, vol. 107, no. 5, August 1987, page 642, abstract no. 39154f,**

**Columbus, Ohio, US; A. HARADA et al.: "Cyclodextrin-palladium chloride. New catalytic system for selective oxidation of olefins to ketones"**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **De Jong, Bartele Sybren**
**Badhuisweg 3**
**NL-1031 CM Amsterdam(NL)**
Inventor: **Stapersma, Johan**
**Badhuisweg 3**
**NL-1031 CM Amsterdam(NL)**
Inventor: **Tomassen, Henricus Paulus Maria**
**Badhuisweg 3**
**NL-1031 CM Amsterdam(NL)**

EP 0 308 031 B1

**Description**

The invention relates to polymeric surfactants, to a process for the preparation of the polymeric surfactants, to derivatives thereof and to the use of the polymeric surfactants and their derivatives.

Many reactions are subject to significant rate and selectivity effects when carried out in the presence of surfactant aggregates such as micelles. A widespread application of these effects however, is thwarted by the often large amounts of surfactants needed to achieve these results and also by the problematic work-up of such reaction mixtures. Moreover, such surfactant aggregates are very sensitive to changes in solvent, temperature and to the presence of salts and other substances. A further restriction in their applicability is the limited number of solvents wherein such surfactant aggregates are formed.

Hence, a considerable improvement would be achieved when, in reactions as hereinbefore described, large amounts of surfactants or high surfactant concentrations would not be required, and also when such surfactant aggregates would be less sensitive to environmental conditions such as solvent type and temperature or to the presence of salts and other substances.

A method for providing surfactant aggregates which can be expected to be less sensitive to the environmental conditions, has been described in J. Polym. Sci., Polym. Lett. Ed., 17(12) 749-51(1979). Said method comprises the radiation-induced polymerization of sodium 10-undecenoate in an aqueous solution, wherein said compound was present in a concentration which exceeded its critical micelle concentration. Although the poly (sodium 10-undecenoate) thus obtained will probably adopt a micellar-like structure when dissolved in an aqueous medium, it can be expected that with this product the micellar-like structure will disappear when e.g. the carboxylate groups are replaced with other groups or when the poly (sodium 10-undecenoate) is dissolved in a different solvent.

The problem underlying the present invention is to provide surfactant aggregates which do not suffer from one or more of the disadvantages hereinbefore described.

There is surprisingly found that this problem can be solved by crosslinked, polymeric surfactants, wherein the starting monomeric surfactants have at least two unsaturated moieties in the hydrocarbon chain forming the hydrophobic part of the surfactant molecule, one of the unsaturated moieties forming a terminal group or containing a methyl or ethyl group at the terminal carbon atom, whilst the polar group of the surfactant molecule is connected to the last carbon atom at the other end of the hydrocarbon chain. Preferably the monomers contain conjugated unsaturated moieties, whereby the unsaturation is preferably alkynic rather than alkenic. Said crosslinked polymeric surfactants are novel compounds.

It is believed that in these polymeric surfactants, typically linkages are present which connect the monomer derived surfactant groups and which do not, or only partly form linear structures. Instead, or in addition thereto, the polymeric surfactants contain crosslinking bonds which contribute to stabilizing the structure of the crosslinked polymeric surfactants.

Such crosslinked polymeric surfactants are thus products having a crosslinked hydrocarbon centre from which hydrocarbon chains protrude carrying a polar group.

The polar group of the surfactant molecules may conveniently comprise an anionic, a cationic, a nonionic or a zwitterionic group.

Preferred monomeric surfactants containing conjugated alkynic moieties are surfactants of general formula

$$R\text{-}C{\equiv}C\text{-}C{\equiv}C\text{-}(CH_2)_m\text{-}C(H)X\text{-}(CH_2)_n\text{-}R^1 \qquad I$$

wherein R is H, $CH_3$ or $C_2H_5$; $R^1$ represents an anionic, a cationic, a nonionic or a zwitterionic group; X is H, Cl, Br, I or $PR^2R^3$ wherein $R^2$ and $R^3$ are the same or different and represent an alkyl or an aryl group which may optionally be substituted; m and n are integers from 0 to 12 included and $m+n$ has a value from 4 to 12 included, with the proviso that the number of carbon atoms in the surfactant molecules of general formula I, including R but excluding $R^1$, does not exceed 17 when X is H.

The present invention further relates to a process for the preparation of crosslinked, polymeric surfactants, wherein a monomeric surfactant as hereinbefore described is polymerized in a polar solvent wherein said surfactant is present in a concentration which is higher than its critical aggregate concentration, as hereinafter defined, at the temperature of polymerization.

In the context of the present invention the term critical aggregate concentration is defined as the concentration of a given surfactant in a given solvent at a given temperature above which concentration said surfactant forms aggregates.

The nature of the aggregates formed is governed amongst others by the type of solvent and the type and concentration of the surfactant, but is as such not critical. The solvents which may conveniently be

2

employed in the process of the present invention are polar solvents wherein a surfactant of general formula I will form aggregates. Aggregate formation and the critical aggregate concentration can conveniently be established e.g. via lightscattering or conductometric methods. Suitable solvents include water, lower mono- and polyhydric alcohols, dimethyl sulfoxide, dimethyl formamide and the like, or mixtures thereof. Water is a preferred solvent.

It is generally accepted that such an aggregate comprises a lipophilic core consisting of the hydrocarbon chains of the surfactant molecules, which core is separated from the solvent phase by an interface in which the hydrophylic groups of the surfactant molecules are located.

It is expected that such a selective orientation of the surfactant molecules in the aggregates, i.e. one wherein the hydrocarbon chains carrying the polymerizable groups are in close contact with one another in the core of the aggregate, will have a beneficial effect on the polymerization of said surfactants.

The polymerization of the surfactants within the aggregates can be effected by conventional polymerization techniques such as in the presence of a free-radical initiator system. When such a free-radical initiator system is used there is a preference for employing an initiator which is insoluble or only partly soluble the solvent used. Such an initiator system can conveniently be introduced into the solvent system together with the surfactant.

A preferred method for the preparation of the polymeric surfactants hereinbefore described, is to employ a surfactant of general formula I and to conduct the polymerization via a photochemically-induced polymerization procedure, for which a $^{60}$Co source or UV irradiation is frequently used.

Preferred anionic groups $R^1$ of the surfactants of general formula I are selected from the group comprising $CO_2Y$, $SO_3Y$, $OSO_3Y$, $(OCH_2CH_2)_pOSO_3Y$ wherein p is an integer in the range of from 1-10, and $CON(CH_3)CH_2CH_2SO_3Y$, wherein Y represents an alkalimetal atom or $NH_4$. Especially preferred are such anionic group containing surfactants wherein Y is a sodium atom.

Examples of suitable cationic groups $R^1$ are quarternary ammonium groups of general formula

$$-N^+(R^4)_3Z^- \qquad (III)$$

wherein each $R^4$ is the same or different and represents a lower alkyl, aryl or an alkaryl group, and Z represents a halogen atom or OH.

Examples of suitable nonionic groups $R^1$ are oligomers of ethylene oxide or propylene oxide as well as oligomers based on mixtures of ethylene oxide and propylene oxide.

Examples of suitable zwitterionic groups $R^1$ are groups of general formula

$$-N^+(R^4)_2(CH_2)_rQ^- \qquad (IV)$$

wherein $R^4$ has the same meaning as in general formula III, r is an integer in the range of from 1 to 5 and Q is a $CO_2Y$ or $SO_3Y$ group, wherein Y has the same meaning as hereinbefore described.

Preferred surfactants are those of general formula I wherein $R^1$ represents an anionic group.

Should the surfactant molecules of general formula I carry a group X, which X ≠ H, it is preferred that said group X is carried by the alpha-C atom of the molecule, i.e. a surfactant of general formula I wherein n is o. Sodium 10,12-tetradecadiynoate is an especially preferred anionic group-containing surfactant.

For certain applications it may be advantageous to conduct the polymerization of the surfactants of general formula I in the presence of one or more cosurfactants. In addition to a polymerizable group it is preferred that such a cosurfactant should have a more or less similar molecular structure as the surfactants of general formula I in order to obtain mixed aggregates. Suitable cosurfactants include surfactants of general formula

$$R^5-(CH_2)_m1-C(H)X-(CH_2)_n1-R^1 \qquad II$$

wherein $R^1$ and X have the same meaning as in general formula I; $R^5$ is $CH_2=CH$, $CH≡C$, $CH_3-C≡C$, $C_2H_5-C≡C$ or $CH_2=CH-CH=CH$; $m^1$ and $n^1$ are integers from 0 to 12 included and $m^1+n^1$ has a value in the range of from 6 to 12 included, with the proviso that the number of carbon atoms in the surfactant molecules of general formula II, including $R^5$ but excluding $R^1$, does not exceed 17, when X is H.

When in the practice of the present invention the polymerization of the surfactants of general formula I is conducted in the presence of one or more surfactants of general formula II, it is preferred that the molar fraction of the surfactants of general formula I comprises at least 50 % mol of the total surfactant composition.

Upon completion of the polymerization, the polymeric surfactants may be isolated by known methods. Suitable methods include evaporation of the solvent medium or precipitation in a non-solvent after first having removed the excess of solvent wherein the polymerization had been conducted. With the latter procedure it may be advantageous to employ a solvent wherein the polymeric surfactant is insoluble while any non-polymerized surfactant and/or the relatively low molecular weight polymeric surfactants are soluble.

An alternative method for isolating such polymeric surfactants having anionic polar groups is one wherein the polymeric surfactant is obtained as the acid derivative thereof. With said method, the solvent medium containing the polymeric surfactant is acidified before being evaporated to dryness. Subsequently the relatively low molecular weight species may, if required, be extracted from the residue using e.g. a hydrocarbon solvent. Finally the residue is dissolved in a solvent system which will enable the removal of the inorganic salts e.g. via filtration, which may be followed by evaporation of the solvent to obtain the polymeric surfactant in the acid form.

The crosslinked polymeric surfactants may be used as such or they can be modified before use.

With the anionic group-containing polymeric surfactants for example, one or more of the alkalimetal or ammonia groups may be replaced with different metals or alternatively the anionic group can be converted e.g. into the corresponding acid chloride or in an ester group. These modifications can be conducted via known methods.

The crosslinked polymeric surfactants or their derivatives as hereinbefore described, can be used in chemical and physical processes e.g. as phase transfer catalysts.

The invention will be further illustrated with the following examples for which the following information is provided:

Sodium 10,12-tetradecadiynoate solution.

A 500 ml 0.075 M solution of sodium 10,12-tetradecadiynoate was prepared by introducing into a 1 l three-necked flask the appropriate amounts of sodium hydroxide, freshly recrystallized (from n-hexane) 10,12-tetradecadiynoic acid and demineralized water and allowing the solution to stand in an argon atmosphere in the dark for 72 hours.

The 10,12-tetradecadiynoic acid was prepared via the coupling of 1-bromopropyne and 10-undecynoic acid following the method as described by G. Englinton and W. McCrae in Adv.Org.Chem. Vol.4, p252-274.

Example I

Preparation of poly (sodium 10,12-tetradecadiynoate)

The three-necked flask containing the 500 ml 0,075 M solution of sodium 10,12-tetradecadiynoate prepared as hereinbefore described, was connected to a falling film photo reactor [Applied Photophysics TFQ 15, equipped with a 15 W low pressure mercury lamp (Applied Photophysics model 3020) and a quartz filter]. The solution was irradiated for 240 hours without applying external cooling to the solution, in the course of which the temperature increased from 20 °C to 30 ± 5 °C. The resulting reddish-brown solution was acidified with 1 N HCl solution and subsequently evaporated to dryness using a rotary film evaporator at 50 °C and 1.33 mbar. The residue was treated with 200 ml boiling hexane to remove any unconverted monomer and subsequently dissolved in 50 ml of a 1:10 v/v blend of methanol and dichloromethane and filtered to remove the inorganic salts. The product was isolated by evaporating the solvents at 50 °C and 1.33 mbar. The isolated poly (10,12-tetradecadiynoic acid) was obtained in a > 90 % wt yield, based sodium 10,12-tetradecadiynoate intake.

The corresponding poly (sodium 10,12-tetradecadiynoate) was obtained by neutralisation of the acid via titration with 0.1 N aqueous NaOH followed by drying.

The product was found to be soluble in:

```
water                                  as the sodium salt
tetrahydrofuran                        in the acid form
methanol                               "    "    "    "
dimethyl formamide                     "    "    "    "
trichloromethane/methanol 95/5 v/v     "    "    "    "
dichloromethane/methanol 95/5 v/v      "    "    "    "
trichloromethane                       as the methyl ester
                                       (obtained via ester-
                                       fication of the acid
                                       with diazomethane)
tetrachloromethane                     as the acid chloride
thionylchloride                        "    "    "      "
                                       (obtained via react-
                                       ion of the acid with
                                       thionylchloride)
```

Molecular weight determination of the methylester of poly (10,12-tetradecadiynoic acid) via gel permeation chromatography, using polystyrene as reference, gave the following results

$\overline{M}n$ = 4930

$\overline{M}w$ = 6950

$$\frac{\overline{M}w}{\overline{M}n} = 1.41$$

The outcome of NMR ($^1$H, $^{13}$C) analysis in different solvents as well as in in the solid state, in combination with negative results of the tests for unsaturation via $Br_2$ and $O_3$, confirm that the products prepared by the process of the present invention are crosslinked compounds.

Example II

Wacker oxidation of 1-hexene in the presence of poly (sodium 10,12-tetradecadiynoate)

0.5 mmol $PdCl_2$, 5 mmol $CuCl_2$, 0.5 mmol of poly (sodium 10,12-tetradecadiynoate) as prepared in example I, 12.5 mmol of 1-heptene, 4.2 mmol n-undecane (as internal standard) and 11 ml water were introduced into a two-necked reactor, equipped with a reflux condensor, and stirred (magnetically) at high speed. The mixture was covered with an oxygen blanket, which was made up by a slow stream of oxygen which entered the reactor just above the surface of the mixture. The reactor was placed in a thermostated oil bath (70° ± 5 °C) and the reaction was allowed to proceed for 4 hours. Subsequently the oxygen stream was stopped, 2.5 ml pentane was added to the reactor via the reflux condensor and the mixture was allowed to reflux for 1 minute followed by cooling to 20 °C.

The product was analysed by gas liquid chromatography and the resulting data are given in Table I.

Comparative experiments A and B.

Following the procedure of example II and simultaneously with this example, two comparative experiments were carried out which only differed from example II in that in experiment A no poly (sodium 10,12-tetradecadiynoate) was used while in experiment B the poly (sodium 10,12-tetradecadiynoate) was replaced with an equal amount of sodium 10,12-tetradecanoate. The results of these experiments are also given in Table 1.

## Table 1

| Example | 1-heptene conversion % mol | 2-heptanone % mol |
|---------|---------|---------|
| I | 38 | 30 |
| A (comp.exp.) | 24 | 19 |
| B ( " " ) | 29 | 22 |

It can be seen that the presence of poly (sodium 10,12-tetradecadiynoate) has a beneficial influence on the conversion of 1-heptene and the formation of 2-heptanone vis à vis the reaction wherein no phase transfer catalyst is used, but is also more effective than the sodium 10,12-tetradecanoate.

**Claims**

1. Crosslinked, polymeric surfactants, wherein the starting monomeric surfactants have at least two unsaturated moieties in the hydrocarbon chain forming the hydrophobic part of the surfactant molecule, one of the unsaturated moieties forming a terminal group, or containing a methyl or ethyl group at the terminal carbon atom, whilst the polar group of the surfactant molecule is connected to the last carbon atom at the other end of the hydrocarbon chain.

2. Polymeric surfactants as claimed in claim 1, wherein the unsaturated moieties are conjugated unsaturated moieties.

3. Polymeric surfactants as claimed in claim 1 or 2, wherein the unsaturated moieties are alkynic unsaturated moieties.

4. Polymeric surfactants as claimed in claim 1-3, wherein the alkynic unsaturated moieties containing surfactants are monomeric surfactants of general formula

    $R-C{\equiv}C-C{\equiv}C-(CH_2)_m-C(H)X-(CH_2)_n-R^1$    I

    wherein R is H, $CH_3$ or $C_2H_5$; $R^1$ represents an anionic, a cationic, a nonionic or a zwitterionic group; X is H, Cl, Br, I or $PR^2R^3$ wherein $R^2$ and $R^3$ are the same or different and represent an alkyl or an aryl group, which may optionally be substituted; m and n are integers from 0 to 12 included and $m+n$ has a value in the range of from 4 to 12 included, with the proviso that the number of carbon atoms in the surfactant molecules of general formula I, including R but excluding $R^1$, does not exceed 17, when X is H.

5. A process for the preparation of crosslinked, polymeric surfactants as claimed in any one of claims 1-4, wherein the monomeric surfactant is polymerized in a polar solvent, wherein said surfactant is present in a concentration which is higher than its critical aggregate concentration, at the temperature of polymerization.

6. A process as claimed in claim 5, wherein the solvent is water.

7. A process as claimed in claims 5 or 6, wherein a surfactant of general formula I is used, and a photochemically-induced polymerization procedure is employed.

8. A process as claimed in any one of claims 5 to 7, wherein in general formula I $R^1$ represents an anionic group.

**9.** A process as claimed in any one of claims 5 to 8, wherein the anionic groups $R^1$ are selected from the group consisting of $CO_2Y$, $SO_3Y$, $OSO_3Y$, $(OCH_2CH_2)_pOSO_3Y$ wherein p is an integer in the range of from 1-10 and $CON(CH_3)CH_2CH_2SO_3Y$ wherein Y represents an alkalimetal atom or $NH_4$.

**10.** A process as claimed in claim 9, wherein Y represents a sodium atom.

**11.** A process as claimed in any one of claims 5-10, wherein in general formula I n is 0 when X ≠ H.

**12.** A process as claimed in any of claims 5 to 11, wherein the surfactant is sodium 10,12-tetradecadiynoate.

**13.** A process as claimed in any one of claims 5-12, wherein the polymerization of the surfactant of general formula I is conducted in the presence of one or more surfactants of general formula

$$R^5\text{-}(CH_2)_m1\text{-}C(H)X\text{-}(CH_2)_n1\text{-}R^1 \qquad \text{II}$$

wherein $R^1$ and X have the meaning as hereinbefore defined; $R^5$ is $CH_2=CH$, $CH\equiv C$, $CH_3\text{-}C\equiv C$, $C_2H_5\text{-}C\equiv C$ or $CH_2=CH\text{-}CH=CH$; $m^1$ and $n^1$ are integers from 0 to 12 included and $m^1+n^1$ has a value in the range from 6 to 12 included, with the proviso that the number of carbon atoms in the surfactant molecules of general formula II, including $R^5$ but excluding $R^1$ does not exceed 17 when X is H.

**14.** Use of crosslinked polymeric surfactants as claimed in any one of claims 1-4, as phase transfer catalyst.

**Patentansprüche**

**1.** Vernetzte oberflächenaktive Polymere, wobei die oberflächenaktiven Ausgangsmonomeren mindestens zwei ungesättigte Teile in der Kohlenwasserstoffkette haben, die den hydrophoben Teil des oberflächenaktiven Moleküls bilden, wobei ein ungesättigter Teil eine Endgruppe bildet oder eine Methyl- oder Ethylgruppe am endständigen Kohlenstoffatom enthält, während die polare Gruppe des oberflächenaktiven Moleküls an das letzte Kohlenstoffatom am anderen Ende der Kohlenwasserstoffkette gebunden ist.

**2.** Oberflächenaktive Polymere wie in Anspruch 1 beansprucht, in denen die ungesättigten Teile konjugierte ungesättigte Teile sind.

**3.** Oberflächenaktive Polymere wie in den Ansprüchen 1 oder 2 beansprucht, in denen die ungesättigten Teile alkinisch ungesättigte Teile sind.

**4.** Oberflächenaktive Polymere wie in den Ansprüchen 1 bis 3 beansprucht, in denen die alkinisch ungesättigten Teile, die die Oberflächenaktivität enthalten, oberflächenaktive Monomere der allgemeinen Formel

$$R\text{-}C\equiv C\text{-}C\equiv C(\text{-}CH_2)_m\text{-}C(H)X\text{-}(CH_2)_n\text{-}R^1 \qquad \text{I}$$

in der R Wasserstoff, $CH_3$ oder $C_2H_5$ ist, $R^1$ eine anionische, eine kationische, eine nichtionische oder eine zwitterionische Gruppe darstellt, X Wasserstoff, Cl, Br, J oder $PR^2R^3$ ist, wobei $R^2$ und $R^3$ gleich oder verschieden sind und eine Alkyl- oder Arylgruppe darstellen, die gegebenenfalls substituiert sein kann, m und n ganze Zahlen von 0 bis 12 einschließlich sind und m+n einen Wert von 4 bis 12 einschließlich hat, mit der Maßgabe, daß die Zahl der Kohlenstoffatome in den oberflächenaktiven Molekülen der allgemeinen Formel I, einschließlich R, jedoch ausgenommen $R^1$, 17 nicht übersteigt, wenn X Wasserstoff ist.

**5.** Ein Verfahren zur Herstellung von vernetzten oberflächenaktiven Polymeren wie in irgendeinem der Ansprüche 1-4 beansprucht, wobei das oberflächenaktive Monomere in einem polaren Lösungsmittel polymerisiert wird, wobei dieses oberflächenaktive Monomere in einer Konzentration vorhanden ist, die höher als seine kritische Aggregatkonzentration bei der Polymerisationstemperatur ist.

6. Ein Verfahren wie in Anspruch 5 beansprucht, wobei das Lösungsmittel Wasser ist.

7. Ein Verfahren wie in Ansprüchen 5 oder 6 beansprucht, wobei ein oberflächenaktives Monomeres der allgemeinen Formel I verwendet wird und eine photochemisch induzierte Polymerisationsarbeitsweise angewendet wird.

8. Ein Verfahren wie in irgendeinem der Ansprüche 5 bis 7 beansprucht, wobei in der allgemeinen Formel I $R^1$ eine anionische Gruppe darstellt.

9. Ein Verfahren wie in irgendeinem der Ansprüche 5 bis 8 beansprucht, wobei die anionische Gruppe $R^1$ aus der Gruppe bestehend aus $CO_2Y$, $SO_3Y$, $OSO_3Y$, $(OCH_2CH_2)_pOSO_3Y$, wobei p eine ganze Zahl im Bereich von 1-10 ist, und $CON(CH_3)CH_2CH_2SO_3Y$ ausgewählt ist, in der Y ein Alkalimetallatom oder $NH_4$ darstellt.

10. Ein Verfahren wie in Anspruch 9 beansprucht, wobei Y ein Natriumatom ist.

11. Ein Verfahren wie in irgendeinem der Ansprüche 5 bis 10 beansprucht, wobei in der allgemeinen Formel I n Null ist, wenn X ungleich H ist.

12. Ein Verfahren wie in irgendeinem der Ansprüche 5 bis 11 beansprucht, wobei das oberflächenaktive Monomere Natrium10,12-tetradecadiinoatist.

13. Ein Verfahren wie in irgendeinem der Ansprüche 5 bis 12 beansprucht, wobei die Polymerisation des oberflächenaktiven Monomeren der allgemeinen Formel I in Gegenwart von einem oder mehreren oberflächenaktiven Monomeren der allgemeinen Formel

$$R^5\text{-}(CH_2)_m 1\text{-}C(H)X\text{-}(CH_2)_n 1\text{-}R^1 \qquad (II)$$

durchgeführt wird, in der $R^1$ und X die Bedeutung wie vorstehend definiert haben, $R^5$ $CH_2=CH$, $CH\equiv C$, $CH_3\text{-}C\equiv C$, $C_2H_5\text{-}C\equiv C$ oder $CH_2=CH\text{-}CH=CH$ ist, $m^1$ und $n^1$ ganze Zahlen von 0 bis 12 einschließlich sind und $m^1 + n^1$ einen Wert im Bereich von 6 bis 12 einschließlich hat, mit der Maßgabe, daß die Zahl der Kohlenstoffatome in den oberflächenaktiven Molekülen der allgemeinen Formel II, einschließlich $R^5$, jedoch ausgenommen $R^1$, 17 nicht übersteigt, wenn X Wasserstoff ist.

14. Verwendung der vernetzten oberflächenaktiven Polymere wie in irgendeinem der Ansprüche 1 bis 4 beansprucht als Phasenübertragungskatalysator.

## Revendications

1. Tensioactifs polymères réticulés, dans lesquels les tensioactifs monomères de départ possèdent au moins deux groupements insaturés dans la chaîne hydrocarbonée formant la partie hydrophobe de la molécule de tensioactif, l'un des groupements insqaturés formant un groupe terminal ou contenant un groupe méthyle ou éthyle sur l'atome de carbone terminal, tandis que le groupe polaire de la molécule de tensioactif est fixée sur le dernier atome de carbone à l'autre extrémité de la chaîne hydrocarbonée.

2. Tensioactifs polymères selon la revendication 1, dans lesquels les groupements insaturés sont des groupements insaturés conjugués.

3. Tensioactifs polymères selon la revendication 1 ou 2, dans lesquels les groupements insaturés sont des groupements insaturés alcyniques.

4. Tensioactifs polymères selon l'une quelconque des revendications 1-3, dans lesquels les tensioactifs contenant des groupements insaturés alcyniques sont des tensioactifs monomères de formule générale

$$R\text{-}C\equiv C\text{-}C\equiv\text{-}(CH_2)_m\text{-}C(H)X\text{-}(CH_2)_m\text{-}R^1 \qquad I$$

dans laquelle R est H, $CH_3$ ou $C_2H_5$ ; $R^1$ représente un groupe anionique, cationique, non ionique ou zwittérionique ; X est H, Cl, Br, I ou $PR^2R^3$, dans lequel $R^2$ et $R^3$ sont identiques ou différents et représentent un groupe alkyle ou aryle, qui peut être éventuellement substitué ; m et n sont des nombres entiers de 0 à 12 inclus et m + n a une valeur dans la gamme de 4 à 12 inclus, à condition que le nombre d'atomes de carbone dans les molécules de tensioactif de formule générale I, y compris R, mais à l'exclusion de $R^1$, ne dépasse pas 17 lorsque X est H.

5. Procédé de préparation de tensioactifs polymères réticulés selon l'une quelconque des revendications 1-4, dans lequel le tensioactif monomère est polymérisé dans un solvant polaire, dans lequel ledit tensioactif est présent à une concentration supérieure à sa concentration critique pour la formation d'agrégats, à la température de polymérisation.

6. Procédé selon la revendication 5, dans lequel le solvant est l'eau.

7. Procédé selon la revendication 5 ou 6, dans lequel on utilise un tensioactif de formule générale I et on emploie un procédé de polymérisation à amorçage photochimique.

8. Procédé selon l'une quelconque des revendications 5 à 7, dans lequel, dans la formule générale I, $R^1$ représente un groupe anionique.

9. Procédé selon l'une quelconque des revendications 5 à 8, dans lequel les groupes anioniques $R^1$ sont choisis dans le groupe constitué par $CO_2Y$, $SO_3Y$, $OSO_3Y$, $(OCH_2CH_2)_pOSO_3Y$, dans lequel p est un nombre entier dans la gamme de 1-10, et $CON(CH_3)CH_2CH_2SO_3Y$, dans lequel Y représente un atome de métal alcalin ou $NH_4$.

10. Procédé selon la revendication 9, dans lequel Y représente un atome de sodium.

11. Procédé selon l'une quelconque des revendications 5-10, dans lequel, dans la formule générale I, n est 0 lorsque X ≠ H.

12. Procédé selon l'une quelconque des revendications 5 à 11, dans lequel le tensioactif est le 10,12-tétradécadiynoate de sodium.

13. Procédé selon l'une quelconque des revendications 5-12, dans lequel la polymérisation du tensioactif de formule générale I est réalisée en présence d'un ou de plusieurs tensioactifs de formule générale

$$R^5-(CH_2)_m1-C(H)X-(CH_2)_n1-R^1 \qquad II$$

dans laquelle $R^1$ et X ont les significations définies cidessus ; $R^5$ est $CH_2=CH$, $CH\equiv C$, $CH_3-C\equiv C$, $C_2H_5-C\equiv C$ ou $CH_2=CH-CH=CH$ ; $m^1$ et $n^1$ sont des nombres entiers de 0 à 12 inclus, et $m^1 + n^1$ a une valeur dans la gamme de 6 à 12 inclus, à condition que le nombre d'atomes de carbone dans les molécules de tensioactif de formule générale II, y compris $R^5$, mais à l'exception de $R^1$, ne soit pas supérieur à 17 lorsque X est H.

14. Utilisation de tensioactifs polymères réticulés selon l'une quelconque des revendications 1-4 comme catalyseurs de transfert de phase.